(19) Europäisches Patentamt
European Patent Office
Office européen des brevets

(11) **EP 3 479 124 B1**

(12) **EUROPEAN PATENT SPECIFICATION**

(45) Date of publication and mention
of the grant of the patent:
**02.04.2025 Bulletin 2025/14**

(21) Application number: **17737244.8**

(22) Date of filing: **30.06.2017**

(51) International Patent Classification (IPC):
**G01N 33/92** (2006.01)   **G01N 33/543** (2006.01)
**C07K 16/18** (2006.01)

(52) Cooperative Patent Classification (CPC):
**C07K 16/18; G01N 33/543; G01N 33/92;**
C07K 2317/33

(86) International application number:
**PCT/EP2017/066380**

(87) International publication number:
**WO 2018/002362 (04.01.2018 Gazette 2018/01)**

(54) **MEASUREMENT OF FABP FOR DIAGNOSIS**

MESSUNG VON FABP ZUR DIAGNOSE

MESURE DE L'ANTIGÈN FABP POUR LA POSE D'UN DIAGNOSTIC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2016 EP 16177366**

(43) Date of publication of application:
**08.05.2019 Bulletin 2019/19**

(73) Proprietors:
• **Randox Laboratories Ltd
Crumlin, Co Antrim, Northern Ireland BT29 4QY
(GB)**
• **Randox Teoranta
Letterkenny, Co. Donegal F94 TV06 (IE)**

(72) Inventors:
• **MCCONNELL, Ivan
Northern Ireland
Antrim BT29 4QY (GB)**
• **FITZGERALD, Peter
Northern Ireland
Antrim BT29 4QY (GB)**
• **LAMONT, John
Northern Ireland
Antrim BT29 4QY (GB)**
• **RICHARDSON, Ciaran
Dungloe
Co Donegal F94 TV06 (IE)**

(74) Representative: **Gill Jennings & Every LLP
The Broadgate Tower
20 Primrose Street
London EC2A 2ES (GB)**

(56) References cited:
WO-A1-00/33083         WO-A1-2009/139632
WO-A2-2005/029088     WO-A2-2007/007129
US-A1- 2008 008 699    US-A1- 2012 322 682
US-A1- 2015 362 494

• ANONYMOUS: "Anti-liver FABP antibody
[L2B10] ab7366", 2007, XP055303933, Retrieved
from the Internet <URL:http://www.abcam.com/
liver-FABP-antibody-L2B10-ab7366.pdf>
[retrieved on 20160920]
• ANONYMOUS: "Monoclonal antibody to human
B-FABP", 1 April 2012 (2012-04-01),
XP055303966, Retrieved from the Internet
<URL:http://www.hycultbiotech.com/downloads/
dl/file/id/799/product/740/hm2300.pdf> [retrieved
on 20160920]

EP 3 479 124 B1

- KAJIURA SATOSHI ET AL: "Establishment and characterization of monoclonal and polyclonal antibodies against human intestinal fatty acid-binding protein (I-FABP) using synthetic regional peptides and recombinant I-FABP", JOURNAL OF IMMUNOASSAY AND IMMUNOCHEMISTRY, DEKKER, NEW YORK, NY, US, vol. 29, no. 1, 1 January 2008 (2008-01-01), pages 19 - 41, XP009125105, ISSN: 1532-1819, DOI: 10.1080/15321810701735005
- VIJAYALAKSHMI AYYAR B ET AL: "Highly sensitive recombinant antibodies capable of reliably differentiating heart-type fatty acid binding protein from noncardiac isoforms", ANALYTICAL BIOCHEMISTRY, ACADEMIC PRESS INC, NEW YORK, vol. 407, no. 2, 15 December 2010 (2010-12-15), pages 165 - 171, XP027354960, ISSN: 0003-2697, [retrieved on 20100807]
- SHUTARO ISHIMURA ET AL: "Circulating Levels of Fatty Acid-Binding Protein Family and Metabolic Phenotype in the General Population", PLOS ONE, vol. 8, no. 11, 20 November 2013 (2013-11-20), pages e81318, XP055304626, DOI: 10.1371/journal.pone.0081318
- M. M.A.L. PELSERS: "Brain- and Heart-Type Fatty Acid-Binding Proteins in the Brain: Tissue Distribution and Clinical Utility", CLINICAL CHEMISTRY, vol. 50, no. 9, 1 September 2004 (2004-09-01), pages 1568 - 1575, XP055073594, ISSN: 0009-9147, DOI: 10.1373/clinchem.2003.030361
- PELSERS M M A L ET AL: "Fatty acid-binding proteins as plasma markers of tissue injury", CLINICA CHIMICA ACTA, ELSEVIER BV, AMSTERDAM, NL, vol. 352, no. 1-2, 1 February 2005 (2005-02-01), pages 15 - 35, XP027648936, ISSN: 0009-8981, [retrieved on 20050201]
- C. E. TEUNISSEN ET AL: "Brain-specific fatty acid-binding protein is elevated in serum of patients with dementia-related diseases : Brain-specific fatty acid-binding protein in dementia", EUROPEAN JOURNAL OF NEUROLOGY, vol. 18, no. 6, 8 December 2010 (2010-12-08), GB, pages 865 - 871, XP055405717, ISSN: 1351-5101, DOI: 10.1111/j.1468-1331.2010.03273.x
- CHAN CANGEL PUI-YEE ET AL: "New trends in immunoassays", ADVANCES IN BIOCHEMICAL ENGINEERING, BIOTECHNOL, SPRINGER, BERLIN, DE, vol. 109, 1 January 2008 (2008-01-01), pages 123 - 154, XP008090180, ISSN: 0724-6145

## Description

### Field of the invention

The present invention relates to diagnosis of disease or injury using fatty acid binding proteins (FABPs).

### Background of the invention

Diagnosis of disease and injury in a patient can be both complex and imperfect and is especially apparent when there is insufficient or no information available from the patient. This can be due to the patient's condition, if, for example, the patient presents at a healthcare facility in an unconscious state or is suffering from dementia, is elderly with poor memory, or, in the case of non-human patients, is incapable of providing information. Confronted with this situation, a medic has to make life-dependent decisions using sub-optimal data.

Even when the patient is able to provide comprehensive information in relation to a disease or injury, multiple diagnoses may still be possible. Following the initial medic-patient interaction, it is common for the patient to undergo further testing, especially blood or urinary biochemical analysis. There is a multitude of possible biochemical tests each providing information, either alone or in combination, of the status of one or more tissues or organs. The medic frequently directs the biochemical testing to a specific organ based on an incomplete diagnosis using sub-optimal information.

A test which could pin-point or help pin-point the specific organ(s) or tissue(s) subject to the disease or injury would be an invaluable initial screening tool and would support a more efficient and accurate diagnostic procedure. This would be especially valuable in a point-of-care format device to facilitate *in situ* diagnosis at, for example, vehicle accident sites.

Fatty acid binding proteins (FABPs) are small cytoplasmic proteins of approximately 15 KDa whose physiological roles facilitate the transport of long-chain fatty acids. They have been researched over several decades and have proven to be useful markers of various injuries and pathologies. They exist in nine isoforms, sharing varying levels of sequence and structural homology (~30-70%) and are found throughout the body. In general, particular isoforms are more abundant in specific tissues (Table 1).

Table 1 Tissue localisation of FABPs in healthy individuals (References: Journal of Nutritional Biochemistry (2010), 21(11) 1015-1032; Human Genomics (2011), 5(3) 170-191; Journal of Biological Chemistry (2010), 285(43) 32682).

| Organ | Fatty Acid Binding Protein | | | | | | | | |
|---|---|---|---|---|---|---|---|---|---|
|  | Liver | Intestine | Heart | Adipocyte | Epidermal | Ileal | Brain | Myelin | Testis |
| Heart |  |  | A |  | B |  |  |  |  |
| Brain |  |  | B |  | B |  | A |  |  |
| Liver | A | C |  |  | B |  |  |  |  |
| Kidney | A |  | A |  | B |  |  |  |  |
| Lungs | C |  | A |  | B |  |  |  |  |
| Skin |  |  |  |  | A |  |  |  |  |
| Intestine | B | A |  |  |  | A |  |  |  |
| Muscle |  |  | A | C | B |  |  |  |  |
| Adipose |  |  |  | A | C |  |  |  |  |
| Stomach | U |  | U |  | U | U |  |  |  |
| Pancreas | A |  |  |  |  |  |  |  |  |
| Testis |  |  |  |  | B |  |  |  | A |
| Ovaries |  |  | U |  |  | U |  |  |  |
| Mam gl |  |  | U |  | U |  | U |  | U |
| Adrenal |  |  | U |  |  | U |  |  |  |
| PNS |  |  |  |  |  |  |  | A |  |
| A = Major; B= Moderate; C = Minor; U = Unknown | | | | | | | | | |

Following tissue insult and cellular damage, FABPs are released rapidly into the interstitium and plasma. In the event of a non-hospital based trauma incident, the ability to administer a comprehensive whole body screen by detecting the full range of FABPs could help inform potentially life-saving management and treatment of the patient. EP2283360 describes a device and method for separating blood and analysing heart FABP, but does not detail or exemplify a device or method for the simultaneous measurement of multiple FABPs. Moreover, existing FABP antibodies display high levels of cross-reactivity between multiple FABP isoforms. This limitation has impeded significantly the development of a point-of-application or point-of-care platform for detecting and discriminating between multiple FABPs to facilitate the accurate and rapid diagnosis of tissue injury.

WO2009/139632 discloses a device and method for separating and analysing blood. The device may be used to detect FABP in a patient blood sample. The binding of FABP with a detection antibody is disclosed, with the binding taking place in a liquid sample prior to contact with a device substrate having a capture antibody attached. The device is primarily used for single FABP detection. If multiple FABPs are to be detected, separate substrates are to be used for each FABP (see figure 3 and page 15), however no example is provided for such.

There is consequently a pressing need to develop new reagents and methods in order to realise the diagnostic potential of FABPs.

## Summary of the invention

Despite the sequence and structural homology between FABP isoforms, the present inventors have surprisingly found that B-FABP, E-FABP, A-FABP, H-FABP, L-FABP, II-FABP, I-FABP, M-FABP and T-FABP each display unique epitopes recognised by antibodies with high specificity and low cross-reactivity. Each antibody targeted towards a specific FABP antigen has been found to display a cross-reactivity of less than 5%.

The present invention therefore describes and exemplifies a multiplexing system, an immunoassay kit and methods based on these antibody technologies for the simultaneous detection and measurement of specific FABPs within samples which may comprise B-FABP, E-FABP, H-FABP and L-FABP. Unlike other analytical techniques, such as chromatography linked to mass spectrometry (GC-MS, LC-MS), this invention enables a comprehensive FABP screen that is applicable in a format that is simple to operate, affordable, and can generate results in 15 minutes.

In accordance with the first aspect of the invention, there is provided a multiplexing system comprising a support substrate having immobilised thereon a binding molecule for each of the following, Brain-Fatty Acid Binding Protein (B-FABP), Liver-Fatty Acid Binding Protein (L-FABP), Heart-Fatty Acid Binding Protein (H-FABP) and Epidermal-Fatty Acid Binding Protein (E-FABP), wherein the binding molecule for each binds specifically to said Fatty Acid Binding Protein (FABP) at the following regions; B-FABP at the region defined by any of SEQ ID Nos. 1 (LGEEFDETTADDRNCKSV) and 2 (DGKMVMTLTFGDVVAVRHYEKA), L-FABP at the region defined by any of SEQ ID Nos. 7 (AFMKAIGLPEELIQKGKDI) and 8 (TNTMTLGDIVFKRISKRI), H-FABP at any of the region defined by any of SEQ ID Nos. 3 (MVDAFLGTWKLVDSKNFDD) and 4 (VKSIVTLDGGKLVHLQKWD), E-FABP at the region defined by any of SEQ ID Nos. 5 (GFDEYMKELGVGIALRKM) and 6 (MATVQQLEGRWRLVDSKGF), wherein the binding molecules specific to each FABP display a cross-reactivity of less than 5% to the other isoforms.

In a second aspect of the invention, there is provided an immunoassay kit for detecting different FABPs comprising the multiplexing system according to the first aspect of the invention, and one or more detection probes capable of binding to said FABPs, said detection probe comprising a detectable label.

In a third aspect of the invention, there is provided an *in vitro* method for detecting or quantifying B-FABP, L-FABP, H-FABP, and E-FABP, within a biological sample, comprising bringing the biological sample into contact with the multiplexing system according to the first aspect of the invention, wherein if binding molecules are immobilised to the support substrate of the multiplexing system, the method further comprises the steps of: i) removing unbound sample, ii) contacting the substrate with a detection probe according to the second aspect of the invention, under conditions to allow the detection probe to bind to any bound FABP, and iii) detecting the binding of the detection probe on the substrate to thereby determine the presence of FABP.

## List of drawings

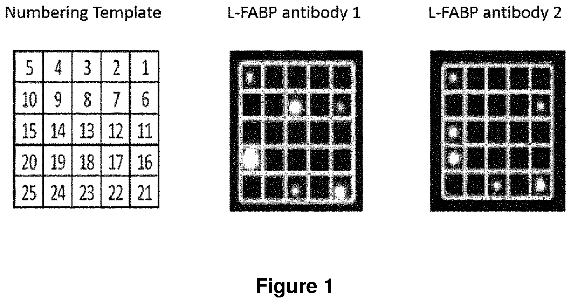

**Figure 1** shows a L-FABP peptide fragment Biochip with 25 discrete target regions to which immobilised are; 1) Blank, 2) Blank, 3) Blank, 4) Blank, 5) Reference spot, 6) Reference Spot, 7) L-FABP residues 1-19, 8) L-FABP residues 17-35, 9) L-FABP residues 33-51, 10) L-FABP residues 49-67, 11) L-FABP residues 65-83, 12) L-FABP residues 81-98, 13) Blank, 14) L-FABP residues 96-112, 15) L-FABP residues 110-127, 16) Blank, 17) BSA, 18) BSA, 19) cTnI peptide-BSA, 20) Full length L-FABP (source 1), 21) Full length L-FABP (source 2), 22) Blank, 23) Reference spot, 24) Blank, 25) Blank. The binding of detection binding molecules to specific DTRs is visualised using horse radish peroxidase.

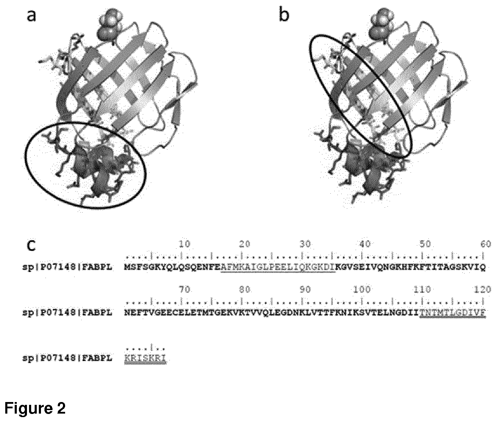

**Figure 2** shows the atomic structure of L-FABP, where the circled structure indicates the binding site of (a) L-FABP antibody 1 and (b) L-FABP antibody 2. The amino acid sequences recognised by L-FABP antibody 1 (single underlined) and L-FABP antibody 2 (double underlined) are shown in relation to the rest of the polypeptide sequence in (c).

## Detailed description of the invention

The present invention provides an effective means for discriminating between different FABP isoforms. The realisation that a family of proteins can display unique epitopes despite sharing such a high degree of structural and sequence homology means that binding molecules with a cross reactivity of less than 5%, preferably less than 1%, can be generated. More specifically, the inventors have shown that raising antibodies against B-FABP, H-FABP, E-FABP and L-FABP that bind to the respective epitopes of SEQ ID No 1 or 2, SEQ ID No 3 and/ or 4, SEQ ID No 5 or 6, and SEQ ID No 7 or 8, generate antibodies that are highly specific and do not display detectable binding to any other FABP family member other than that against which they are raised. The immobilisation of these binding molecules to an easy-to-operate multiplexing system will allow the presence, absence or amount of multiple FABPs within a sample to be determined simultaneously, unambiguously, and rapidly.

The kits, systems, binding molecules, and methods of the present invention will allow for the first time the patterns of FABP antigens within biological samples to be used as an initial screen to determine regions of tissue damage at the point-of-care. Such a comprehensive FABP screen will significantly enhance the speed and efficiency by which medics can administer potentially life-saving treatments following tissue insult and cellular damage. The device may also be used as a companion diagnostic to differentiate between particular diseases that present similar symptoms but exhibit distinct patterns of FABP antigens within biological samples.

In order that the present invention may be more readily understood, the following definitions (taken and modified from the Uniprot database http://www.uniprot.org/uniprot) for the nine exemplified FABPs apply. Additional definitions are set forth throughout the detailed description.

### Liver FABP (FABP1) *Uniprot - P07148*

Liver-type FABP (L-FABP/FABP1) is a ~14kDa member of the fatty acid binding family of proteins. Although predominantly expressed in hepatocytes of the liver, L-FABP is also found in proximal tubular cells of the kidney and in colonocytes and enterocytes (jejunal and ileal) of the gastrointestinal tract. L-FABP has been sequenced as a 127 amino acid polypeptide which contains a lipid binding domain that binds large lipid molecules and their CoA derivatives. The use of L-FABP as a blood biomarker for liver damage has been investigated. Release of cytoplasmic proteins from damaged hepatocytes into the vascular system can be caused by, among other things, acetaminophen (paracetamol) intoxification, ischemia and reperfusion injury, liver congestion, shock, trauma or rejection after transplantation. The hepatocytes are in close contact with the circulatory system, from which they are separated by a single endothelial layer with large clefts. L-FABP release into circulation has been observed following hepatocellular injury due to rejection in a group of liver transplant recipients who had episodes of acute hepatocellular rejection during their post-transplantation stay in the hospital. This study showed that circulatory L-FABP rises significantly in all rejection periods and can be detected in plasma. It has also been shown that both serum and urine levels of L-FABP could be used for diagnosis of chronic kidney disease (CKD). Therefore, the usefulness of L-FABP as a biomarker may not be limited to liver damage. The kidney has been shown to express two types of FABP: H-FABP, located in the distal tubular cells, and L-FABP, which is located in the proximal tubular cells. Urine levels of L-FABP have been shown to be useful in not only diagnosing CKD but also in predicting development of diabetic nephropathy in diabetic patients or progression of disease in non-diabetic patients. Measuring L-FABP levels has also been shown to be useful in monitoring the efficacy of therapeutic drugs for CKD in both diabetic and non-diabetic patients.

L-FABP is also expressed in the gastrointestinal tract along with the intestinal FABP (I-FABP) and may therefore be useful for the early detection of intestinal injury due to small bowel ischemia, trauma, inflammation, or transplant rejection. Intestinal cells that express L-FABP and I-FABP are mainly in the villi of the intestinal wall. When intestinal ischemia is limited to a period of less than 2 h, only the villi are affected and there is a rapid recovery of function. Other studies have investigated the tissue distribution and concentration of I-FABP and L-FABP in surgery and autopsy samples taken along the duodenal to colonal axis of the human intestine. The L-FABP tissue contents in the small intestine were markedly higher than those of I-FABP. Elevated plasma concentrations of both I-FABP and L-FABP have been found in patients suffering from intestinal diseases and severe abdominal injury, while only L-FABP was increased in cases of hepatocellular injury. Therefore, both proteins may serve as early plasma markers of intestinal ischemia.

I-FABP and L-FABP also have potential as diagnostic markers for necrotic enterocolitis in preterm infants; a recent study concluded that L-FABP is a promising sensitive marker for stage I necrotic enterocolitis, while I-FABP appears to be a specific parameter for the early detection of intestinal injury leading to severe stage III necrotic enterocolitis.

Intestinal FABP (FABP2) *Uniprot - P12104*

Intestinal fatty acid binding protein (I-FABP/FABP2) is a 15kDa protein expressed primarily in the enterocytes of the small intestine. Due to its localised expression within the small intestine, I-FABP (in combination with L-FABP, as described above) is an ideal candidate marker for intestinal damage. Several studies have shown that serum/plasma/urine levels of I-FABP are increased in disorders known to result in intestinal damage, such as enterocolitis, intestinal ischemia, ulcerative colitis, celiac disease, abdominal injury and other gastrointestinal complications. As well as this, I-FABP has also been shown to be up regulated in acute pancreatitis. Therefore, I-FABP may have utility in diagnosis and/or monitoring patients with these disorders.

Heart FABP (FABP3) *Uniprot - P05413*

Heart-type fatty acid binding protein (H-FABP/FABP3) is a 15 kDa non-enzymic lipid-binding protein. H-FABP is found in abundance in heart muscle. It is also found in skeletal and smooth muscle, adrenal glands, testes, sebaceous glands, lung, intestine, urogenital tract, lactating mammary gland, kidney, the brain and placenta.

As a result of myocyte injury, H-FABP is released into the circulation where elevated levels are observed within 3 hours of damage, typically returning to baseline values within 12-24 hours. Elevated H-FABP provides a sensitive and specific marker for early diagnosis (or exclusion of) acute myocardial infarction (AMI). H-FABP has also been found to have greater diagnostic potential for Acute Coronary Syndrome than either CK-MB or Troponin I. Clinically, elevation of H-FABP in the circulation indicates historical or on-going cardiomyocyte damage, and is a useful marker for monitoring and managing therapy in hypertension, chronic heart failure, hypertrophic cardiomyopathy, congestive heart failure and pulmonary embolism. H-FABP levels may also prove a useful marker in predicting future cardiac event risk, and be useful for monitoring myocardial damage induced by radiofrequency ablation of arrhythmias and coronary angioplasty. Prolonged elevation of circulatory H-FABP is also observed after acute ischaemic stroke onset, with levels being linked to clinical severity. H-FABP may thus prove a useful marker for stroke damage. Its use determining between different forms of dementia has also been investigated.

H-FABP has also been found to be expressed in the distal tubule of the kidney and has therefore been suggested as a potential marker for i) tubular damage in renal disease patients, ii) patients exposed to heavy metal toxicity and, iii) in donor kidneys prior to transplantation. H-FABP has been shown to be a reliable urinary marker of nephropathy in diabetic patients, an increase in H-FABP being related to albuminuria stage.

Adipocyte FABP (FABP4) *Uniprot - P15090*

Adipose FABP (A-FABP/FABP4), also known as adipocyte P2 or adipocyte protein (aP2), accounts for ~6% of total cellular proteins and is considered to be an adipocytokine due to its expression predominately in adipocytes. It is also co-expressed with Epidermal-FABP (E-FABP/FABP5) in macrophages. Studies have also shown A-FABP is expressed at low levels in dendritic cells, endothelial cells, bronchial epithelial cells, lung, and ovary, spleen, T cell, keratinocyte and tumours. In adipocytes, A-FABP has numerous functions, including regulating insulin secretion during lipolysis, acting as a fatty acid sensor through its interaction with JAK2 (Janus kinase 2 - protein involved in signalling by interferon receptors), and regulating lipid metabolism and differentiation. In macrophages, A-FABP regulates inflammatory responses and cholesterol ester accumulation.

Circulatory levels of A-FABP are increased in overweight/obese individuals, correlating positively with other factors associated with metabolic syndrome, such as increased waist circumference, increased blood pressure, and dyslipidaemia and increased fasting insulin. A-FABP levels have also been shown to correlate with non-alcoholic fatty liver disease (NAFLD) in apparently healthy patients and in diabetic patients. Increases in serum A-FABP have also been linked to several other disorders, including preeclampsia, obstructive sleep apnea (OSAS), renal dysfunction, Cushing's syndrome, atherosclerosis and breast cancer.

Epidermal FABP (FABP5) *Uniprot - Q01469*

Epidermal FABP (E-FABP/FABP5) is also known as PA-FABP (psoriasis-associated FABP) and mal1. As with the other FABP family members, it possesses a tertiary structure of two orthogonal beta-barrels and a basic helix-loop-helix domain adjacent to the ligand binding site. E-FABP is predominately expressed in epidermal cells of the skin, but is also present in other tissues, including the tongue, adipocytes, macrophages, dendritic cells, mammary gland, brain, liver, kidney, lung, testis, skeletal muscle, retina, lens and spleen. In the human lung E-FABP is found in TII cells, macrophages, Clara cells, Goblet cells and endothelial cells. E-FABP is expressed in peripheral neurons during nerve regeneration and is found at high levels in central neurons during neuronal migration and development. A-FABP and E-FABP are co-expressed in macrophages and adipocytes and are linked to the progression of metabolic diseases such as diabetes, obesity and atherosclerosis. Numerous studies have also reported the role of E-FABP in in the development of several cancers including skin, liver, pancreatic, bladder, prostate and breast cancers.

Ileal FABP (FABP6) *Uniprot - P51161*

The ileal fatty acid binding protein (IL-FABP/FABP6), also known as gastrotopin, ileal lipid binding protein (ILBP) or intestinal bile acid binding protein (I-BABP), is approximately 15kDa in size and consists of 128 amino acids. IL-FABP is expressed principally in the ileum, where it is thought to be essential in bile acid reabsorption and intracellular trafficking. IL-FABP preferentially binds to long chain fatty acids, bile acid or retinoids and shares many functional and structural characteristics with L-FABP. As with other FABPs, its small size, tissue specificity and abundance in expression has identified it as a useful marker of cellular damage due to infection, disease, toxicity or trauma. It has been shown that plasma and urinary levels of IL-FABP are increased in patients with intestinal ischemia in line with other markers of intestinal damage (L-FABP and I-FABP). IL-FABP was also shown to be increased in serum of healthy men following exercise, an effect which was proposed to be attributed to splanchnic hypoperfusion. Mutations in IL-FABP have also been shown to be associated with metabolic disorders, such as type 2 diabetes. Finally, increased circulatory IL-FABP has also been associated with various forms of cancer.

Brain FABP (FABP7) *Uniprot - 015540*

Brain-specific FABP (B-FABP/FABP7) was first identified in the brains of rodents where it was shown to be primarily expressed in radial glia. A brain-specific FABP was later isolated and sequenced in adult and foetal human brain tissue. This 132 amino acid protein was found at higher levels in foetal tissue than in adult tissue and therefore it has been presumed to be heavily involved in development. B-FABP is also found in a lesser extent in skeletal muscle. In the adult brain, B-FABP is shown to have a heterogeneous distribution in the adult brain, with relatively high levels in the frontal lobe, temporal cortex and cerebellum. B-FABP has a high affinity for docosahexaenoic acid (DHA) and arachidonic acid (AA), and displays a 4-fold greater affinity to DHA than to AA. Increases in serum levels of B-FABP have been linked to brain injury through trauma, or neurodegenerative disease, such as Alzheimer's and Parkinson's disease. It has also been postulated that due to the differences between B-FABP and H-FABP expression in specific brain regions, calculating the serum ratios of B-FABP/H-FABP may allow for identification of the site of brain injury. Circulating B-FABP has also been considered as a potential early marker of ischaemic stroke and has been detected in the serum of patients as early as two hours following a stroke. Unlike other members of the FABP family, serum levels of B-FABP are virtually undetectable in healthy control subjects, and despite being present in skeletal muscle, it is unknown how damage to these tissues through exercise or disease affects serum levels of the protein. B-FABP has also been shown to play a role in tumour infiltration in malignant gliomas. High levels of B-FABP have also been found in brain tumour tissue compared to normal adult brain. Levels of B-FABP have been shown to correlate with decreased survival times and/or tumour progression in patients suffering from astrocytomas and other malignancies, including melanoma, basal-type breast cancer, and renal cell carcinoma. B-FABP shares an identical DNA sequence to the Mammary-derived Growth Inhibitor-related Gene (MRG) a protein expressed abundantly in the mammary gland which is associated with inhibition of cancer growth and positive prognosis.

Myelin FABP (FABP8) *Uniprot - P02689*

Myelin FABP (M-FABP), also known as FABP8, P2 and PMP2, is found in Schwann cells, probably on the cytoplasmic face of the plasma membrane where it may contribute to fatty acid transport across myelin. Functionally, M-FABP has a high affinity for U-shaped fatty acids such as oleic and palmitic acid. Human M-FABP is 132 amino acids (aa) in length and exhibits two layers of antiparallel β-strands that envelope a hydrophobic pocket for lipid binding. M-FABP is localised specifically in myelinating cells such as Schwann cells of the peripheral nervous system (PNS) and oligodendrocytes in the central nervous system (CNS), where it is found in the compact myelin sheaths that insulate axons. M-FABP constitutes up to 15% of the total protein content within PNS myelin. It is also present in small amounts in CNS myelin, especially in the spinal cord and brain stem myelin. The amount of M-FABP, however, varies between different species, different regions of the nervous system and from fibre to fibre. M-FABP is thought to stabilise the myelin membranes through two opposing positively charged regions on the protein which may facilitate the anchoring of the intracellular membranes. It has also been proposed that M-FABP might function in myelin assembly and turnover. Fragments of M-FABP are known to cause infammatory demyelination in experimental allergic neuritis (EAN), an animal model of Guillain-Barrè syndrome (GBS). Also, antibodies to full length M-FABP and fragments thereof have been found in the serum of GBS patients. These antibodies are predicted to be directly involved in demyelination in a small proportion of GBS cases. However, the presence of antibodies to this intracellular protein is suggestive that myelin damage results in the release of M-FABP into extracellular regions where an acquired immune response can develop. Chronic infammatory demyelinating polyradiculoneuropathy (CIDP), assumed to be the chronic counterpart of acute GBS, is an acquired immune-mediated infammatory disorder of the PNS, with a probable autoimmune pathogenesis. In CIDP, M-FABP was found to be the antigen most likely involved in the immune responses due to nerve damage.

Testis FABP *(FABP9) Uniprot - Q0Z7S8*

Testis fatty acid binding protein (T-FABP/FABP9), also known as PERF15 is found mainly in midpachytene spermatocytes and round spermatids, and constitutes part of the perinuclear theca. Functionally, T-FABP is likely to link intracellular membranes, and may signal abnormal sperm formation during spermatogenesis. Human T-FABP is 132 amino acids (aa) in length and exhibits two layers of antiparallel strands that envelope a hydrophobic pocket for lipid binding. Due to its similarity with M-FABP, it is thought that it functions in lipid membrane anchoring in sperm. There are no commercially available assays for the determination of T-FABP in any sample type. There are also no clinical research studies which have investigated the utility of T-FABP in the diagnosis or monitoring of any disorder. Its presence in testicular tissue, may lend to a potential use in diagnosing testicular cancers or damage to the testis through trauma. It may also have potential uses in monitoring fertility.

The term "binding molecule" herein refers to any molecule that can be generated to recognise a particular target. A non-limiting example of a binding molecule is an aptamer, which may refer to a ribonucleic acid (RNA), deoxyribonucleic acid (DNA) or non-natural nucleic acid (XNA) molecule that typically consists of a short strand of oligonucleotides, or a peptide, consisting of a short sequence of amino acids that can be covalently attached to a protein scaffold in such a way that the aptamer is presented outward from the surface of the scaffold. Suitable engineered and non-engineered non-antibody peptide display scaffolds are known to those skilled in the art. In the context of the present invention, the term "binding molecule" also encompasses antibodies. Binding molecules can be generated to bind to an antigen using any one of a number of well-established techniques known to the skilled person, including, but not limited to, phage display, ribosome display, yeast display, immunization, or hybridoma screening. The preferred binding molecule used in the invention is an antibody.

The term "antibody" refers to an immunoglobulin which specifically recognises an epitope on a target, as determined by the binding characteristics of the immunoglobulin variable domains of the heavy and light chains ($V_H$s and $V_L$s), more specifically the complementarity-determining regions (CDRs). Many potential antibody forms are known in the art, and are included within the above definition. These may include, but are not limited to, a plurality of intact monoclonal antibodies or polyclonal mixtures comprising intact monoclonal antibodies, antibody fragments (for example $F_{ab}$, $F_{ab}$', $F_{(ab')_2}$ and $F_v$ fragments, linear antibodies, single chain antibodies and multispecific antibodies comprising antibody fragments), single chain variable fragments (scF$_v$s), multispecific antibodies, chimeric antibodies, humanised antibodies and fusion proteins comprising the domains necessary for the recognition of a given epitope on a target. An antibody may comprise $\gamma$, $\delta$, $\alpha$, $\mu$ and $\varepsilon$ type heavy chain constant domains, wherein an antibody comprising said domains is designated the class IgG, IgD, IgA, IgM or IgE respectively. Classes may be further divided into subclasses according to variations in the sequence of the heavy chain constant domain (for example IgG1-4). Light chains are designated either $\kappa$ or $\lambda$ class, depending on the identity of the constant region. Antibodies may also be conjugated to various labels that enable detection, including but not limited to radioactive markers, DNA, RNA, fluorescent molecules, or an enzyme which converts a substrate such that its absorbance or chemiluminescence can be detected. Antibodies may also be modified to enable their immobilisation within a substrate in a specific or non-specific orientation.

The term "epitope" refers to the portion of a target which is specifically recognised by a given binding molecule. In instances where the antigen is a protein, the epitope may be formed from either a contiguous or non-contiguous number of amino acids ('linear' or 'conformational' epitopes, respectively), whereby in the case of the latter, residues comprising the epitope are brought together in the three-dimensional fold of the polypeptide. An epitope typically comprises, but is not limited to, 3-10 amino acids in specific positions and orientations with respect to one another. Techniques known in the art for determining the epitope recognised by a binding molecule (specifically whether or not an epitope comprises a given residue) include but are not limited to, site-directed mutagenesis, or the use of suitable homologous proteins to the target protein in combination with techniques for determining specific recognition or lack thereof, as exemplified below. By way of example and not limitation, an epitope may be determined as comprising a given residue by comparative analysis with a control comprising specific recognition of the native (non-substituted) target protein by said antibody; wherein diminished binding and/or lack of specific recognition by said binding molecule when compared with said control identifies a given residue as forming part of an epitope. Furthermore, structural analyses of binding molecule-target protein complexes via x-ray crystallography and/or nuclear magnetic resonance (NMR) spectroscopy, or suitable derivatives thereof, may also be used to determine the residues which constitute an epitope.

As used herein, the term "binds specifically" or "binding specifically", in the context of binding molecule-epitope interactions, refers to an interaction wherein the binding molecule and epitope associate more frequently or rapidly, or with greater duration or affinity, or with any combination of the above, than when either binding molecule or epitope is substituted for an alternative substance, for example an unrelated protein. Generally, but not necessarily, reference to binding means specific recognition. Preferably, references to specific binding in the context of the present invention refer to binding molecules that only bind to the antigen against which they were generated. Techniques known in the art for determining the specific binding or lack thereof to an antigen by another binding molecule include, but are not limited to, FACS analysis, immunocytochemical staining, immunohistochemistry, western blotting/dot blotting, ELISA, affinity chromatography. By way of example and not limitation, specific binding, or lack thereof, may be determined by comparative analysis with a control comprising a binding molecule which is known in the art to specifically recognise said target, or with a control binding molecule characterised by the absence of, or minimal, specific recognition to said target (for example wherein the control comprises the use of a non-specific binding molecule). Said comparative analysis may be either qualitative or quantitative. It is understood, however, that a binding molecule or binding moiety which demonstrates exclusive specific recognition of a given target is said to have higher specificity for said target when compared with a binding molecule which, for example, specifically recognises both the target and a homologous protein.

The term "cross-reactivity" refers to a binding molecule recognising an epitope displayed on an antigen that is not the antigen against which it was raised. For example, a binding molecule generated to bind specifically to A-FABP may cross-react with B-FABP, manifesting in detectable binding to B-FABP to a greater, similar, or lesser extent than to A-FABP. Cross-reactivity can be determined using any of the specific binding assays described previously. Cross-reactivity may also be determined by employing a competition assay. Competition assays measure the ability of a binding molecule to bind to the target antigen upon incubation with an excess of a cross-competing binding molecule or antigen. Cross-reactivity (%CR) may be expressed as a percentage by using to the following equation:

$$\%CR \ = \left(\frac{(OC_s - OC_u)}{TC_s}\right) \times 100$$

wherein $OC_s$ is the total protein concentration bound to a binding molecule when said binding molecule is incubated with the target antigen and an excess of a competing antigen, $OC_u$ is the total protein concentration bound to a binding molecule when said binding molecule is incubated with the target antigen in the absence of competing antigen, and $TC_s$ is the total protein concentration of the target and competing antigen used to determine $OC_s$. Preferably, in the context of the present invention, the binding molecules raised against a particular FABP antigen will display less than 5% cross-reactivity, preferably less than 1% cross-reactivity, to any other FABP family member.

As used herein, the terms "biological sample" and "sample" includes samples obtained from a patient or subject, which may comprise blood, plasma, serum, urine, saliva, tissue extracts, or sputum. In addition, a biological sample may refer to a sample derived from an *in vitro* source, for example, a cell culture suspension, cell lysate, or cell culture supernatant.

The terms "immunoassay", "immuno-detection" and "immunological assay" are used interchangeably herein and refer to antibody-based techniques for identifying the presence of or levels of a protein in a sample. Examples of such assays and methods are well known to those of skill in the art.

As used herein, the term "probe" refers to a binding molecule that is capable of binding and reporting the presence or absence of an antigen within a sample. Probes may be "capture" and "detection" pairs, wherein each probe binds specifically to a distinct epitope upon the target antigen. Capture probes may be engineered in such a way to enable easy separation of the target antigen from a complex sample milieu. For example, capture probes may be engineered to enable their immobilisation to a support substrate, thus enabling off-target molecules to be removed by washing of the support substrate. Capture probes may be immobilised covalently to a support substrate via direct conjugation, or non-covalently by binding of the probe to a molecule on the support substrate that displays a high affinity for an epitope displayed by the capture probe. Detection probes may further comprise detectable labels that can be visualised once a binding event has occurred. Non-limiting examples of detectable labels include radionucleotides, fluorophores, dyes, or enzymes that convert a substrate such that its absorbance or chemilumescence signal can be detected. Probes that may be used in the present invention include but are not limited to antibodies, or single chain variable fragments thereof, aptamers and oligonucleotides. The capture and detection probes for each FABP antigen may display a cross-reactivity of less than 5%, more preferably less than 1%. Alternatively, the detection or capture probe may recognise a universal epitope displayed by all FABP antigens or a subset of FABP antigens. It is appreciated that capture probes and detection probes can be are interchangeable, and the terms simply refer to a pair of binding molecules that can be used together in order to detect the presence of an antigen. Thus, a binding molecule referred to as detection probe is capable of acting as a capture probe, and a binding molecule referred to as a capture probe can act as a detection probe.

The binding molecules of the present invention or FABPs, or peptide fragments thereof, are immobilized to a multiplexing system comprising one or more support substrates. The support substrate may comprise a solid-state device, such as a planar surface, bead, or microparticle, upon which is immobilised a binding molecule that binds specifically to a FABP, or upon which is immobilised FABP, or a peptide fragment thereof. Such binding molecules or FABPs may be immobilised at discrete areas of an activated surface of the support substrate. Alternatively, individual binding molecules that bind specifically to particular antigens, or individual FABPs, may be immobilised to discrete support substrates, wherein the discrete support substrates are combined to form a multiplexing system. The solid-state device may perform multi-analyte assays such that the level of multiple target FABPs in the sample isolated from the patient are determined in parallel. In this context, the support substrate may be one that is used conventionally in multi-analyte microarray technologies. It may, for example, be a biochip, glass slide or other conventional planar support material, or bead or microparticle. The support substrate may be defined as a Discrete Test Area (DTA), which defines the whole substrate, e.g. a single biochip is a DTA. DTAs are physically distinct areas between which liquid or sample flow is not possible. Within each DTA, there may be a plurality of Discrete Test Regions (DTRs, as referred to herein as discrete reaction zones) present. These define discrete locations on a substrate and support binding molecules, or FABPs, or peptide fragments thereof. Each DTR is spatially separated from other DTRs, and each may be used for the same or different reactions, depending on how the reactions are to be performed. The DTRs are usually present within a "biochip", and multiple biochips may be present on the device, each biochip being physically separated from other biochips. In this embodiment, the solid-state device has a multiplicity of DTRs each bearing a desired binding molecule (capture probe) covalently bound to the substrate, and in which the surface of the substrate between the DTRs is inert with respect to the target antigen under study. The solid-state, multi-analyte device may therefore exhibit little or no non-specific binding. Different binding molecules or FABPs may be located in spatially separate locations i.e. within DTRs on the DTA or biochip. In a particular example, the DTA is approximately 1cm$^2$ and there may be $4\times4$ DTRs present within each DTA, preferably $5\times5$ DTRs, $7\times7$ DTRs, $8\times8$ DTRs, $9\times9$ DTRs, $10\times10$ DTRs, $12\times12$ DTRs, $15\times15$ DTRs, $20\times20$ DTRs, $30\times30$ DTRs or greater present within each DTA. Alternatively, the multiplexing system may comprise two or more discrete support substrates, each support substrate supporting ligands which bind specifically to a single FABP isoform or where each discrete support substrate supports an individual FABP, or peptide fragment thereof. The support substrates can be any solid matter which can support FABP-binding molecules, FABPs or peptide fragments thereof. Non-limiting examples of such are beads, microparticles etc. Thus a multiplexing system in the context of the current multi-FABP detecting invention includes any system capable of the concurrent or simultaneous binding and/or detection of two, three, four, five, six, seven, eight or nine, or more FABP isoforms. 'Concurrent' means occurring within a similar time frame this time frame is usually within 30 minutes, preferably within 15 minutes, more preferably within 5 minutes. The concurrent or simultaneous binding and detection of analytes is one of principal advantages of a multiplex analysis system.

In one embodiment, each DTR within a DTA may contain immobilized thereto a binding molecule (capture probe) specific for a distinct FABP antigen. The antigen specificity of each binding molecule immobilized to each DTR upon the DTA may be known. The reactions for determining the presence of different FABPs within the sample isolated from the patient may therefore be carried out at the same time on the same DTA. In this embodiment, a detection probe that recognizes a universal epitope amongst all FABP antigens may be used. Alternatively, detection probes that discriminate between different FABPs may be: i) added to the DTA simultaneously: ii) added to the DTA sequentially: or iii) added to separate DTAs to which capture probes are immobilized and the sample has been contacted to. Where the antigen specificity of capture probes immobilized to each DTR is known, the detection probes may be labeled with the same detectable label. Alternatively, each FABP-specific detection probe may be labeled with a unique detectable label. The presence of FABP antigens within the sample may therefore be determined by detecting the presence of the unique detectable labels attached to each detection probe.

In one embodiment, each DTR within a DTA may contain immobilized thereto a distinct FABP isoform, or peptide fragment thereof (antigen). Thus, a competition assay can be employed in order to determine the presence of multiple antigens within the biological sample. In this embodiment, the biological sample to be analysed may be incubated with detection probes, such as those previously defined, prior to, at the same time, or after the biological sample has been applied to the DTA. The principle of the competition assay dictates that antigens present within the biological sample will "compete" with the antigens immobilized to the discrete DTRs for binding to the detection probes. After a sufficient time to allow binding events to occur, the biological sample can be removed from the DTA by washing and the binding between the detection probes and immobilized antigens at discrete DTRs can be visualized. The degree of binding can inform as to the presence or concentration of the antigen within the biological sample. The more antigen within the sample, the less binding is visualized at discrete DTRs upon which that antigen is immobilized.

A multiplexing substrate of the present invention may be prepared by activating the surface of a suitable substrate, and applying an array of binding molecules (capture probes), or FABPs, or peptide fragments thereof, on to discrete sites on the surface. If desired, the other active areas may be blocked. The binding molecules, FABPs, or peptide fragments thereof, and blocking agents may be bound to the substrate via a linker. In particular, it is preferred that the activated surface is activated using an organosilane or polymer coating before reaction with the binding agent. The solid-state device used in the methods of the present invention may be manufactured according to the method disclosed in, for example, GB-A-2324866 the contents of which is incorporated herein in its entirety. Preferably, the solid-state device used in the methods of the present invention is the Biochip Array Technology system (BAT) (available from Randox Laboratories Limited). More preferably, the Evidence, the Evidence Evolution, Evidence Investigator and Multistat apparatus (available from Randox Laboratories) may be used to determine the levels of biomarkers in the sample.

The multiplexing system of the present invention may further provide quantitative information as to the differences between the concentrations of antigens within a sample as compared to a control. For example, antigen levels within a sample derived from a subject suspected of having multiple tissue damage and/or specific organ failure may be compared to the antigen levels within a control. The control may be a biological sample derived from a subject known not to have undergone any tissue damage and/or specific organ failure. Alternatively, the control may be the average concentration of each antigen derived from a cohort of samples obtained from subjects known not to have undergone any tissue damage or specific organ failure. Means for converting chemical, fluorescent, or radioactive signals originating from the binding of a probe to a target antigen are well known to the skilled artisan. By way of example, and not limitation, antigen concentrations within a sample may be calculated from a calibration curve constructed from a series of known concentrations of protein standards. Preferably the standards are detected using the same detectable label as is present on the FABP detection probe(s). Preferably, the calibration curve is constructed at the same time on the same DTA. By way of example and not limitation, the calibration curve may be constructed from a series of known concentrations of a protein standard.

According to a first aspect, the present invention provides a multiplexing system comprising a support substrate having immobilised thereon a binding molecule for each of the following, Brain-Fatty Acid Binding Protein (B-FABP), Liver-Fatty Acid Binding Protein (L-FABP), Heart-Fatty Acid Binding Protein (H-FABP) and Epidermal-Fatty Acid Binding Protein (E-FABP), wherein the binding molecule for each binds specifically to said Fatty Acid Binding Protein (FABP) at the following regions; B-FABP at the region defined by any of SEQ ID Nos. 1 (LGEEFDETTADDRNCKSV) and 2 (DGKMVMTLTFGDVVAVRHYEKA), L-FABP at the region defined by any of SEQ ID Nos. 7 (AFMKAIGLPEELIQKGKDI) and 8 (TNTMTLGDIVFKRISKRI), H-FABP at any of the region defined by any of SEQ ID Nos. 3 (MVDAFLGTWKLVDSKNFDD) and 4 (VKSIVTLDGGKLVHLQKWD), E-FABP at the region defined by any of SEQ ID Nos. 5 (GFDEYMKELGVGIALRKM) and 6 (MATVQQLEGRWRLVDSKGF), wherein the binding molecules specific to each FABP display a cross-reactivity of less than 5% to the other isoforms.

In a further preferred embodiment, each binding molecule, protein, or peptide fragment thereof is immobilised to a separate support substrate or on discrete areas of the same support substrate. The bead may be a magnetic or paramagnetic bead.

In a further preferred embodiment, the support substrate is a bead or microparticle or a planar surface comprising a plurality of discrete reaction zones, preferably wherein each different binding molecule, or FABP, or peptide fragment thereof, is immobilised upon a separate support substrate or a discrete reaction zone upon a substrate, or wherein each antibody, or fragment thereof, is immobilised upon a discrete reaction zone upon the same support substrate.

The binding molecules specific to each FABP may display a cross-reactivity of less than 5%, preferably less than 1% to the other FABP isoforms. Preferably, the B-FABP binding molecule binds to of SEQ ID No 1 or 2, the H-FABP binding molecule binds to SEQ ID No 3 and/ or 4, the E-FABP binding molecule binds to SEQ ID No 5 or 6, and the L-FABP binding molecule binds to SEQ ID No 7 or 8. More preferably, each binding molecule binds to at least 3 amino acids, preferably 3-7 amino acids, most preferably 3-5 amino acids from the corresponding SEQIDs listed below. The epitopes identified on (SEQ IDs no 1-8) are not intended to limit the invention to binding molecules that exclusively recognise these epitopes. The binding molecules of the present invention encompass all those that bind epitopes upon FABPs that enable their discrimination with a cross-reactivity of less than 5%, preferably less than 1%.

In a preferred embodiment, the binding molecules are antibodies, or single chain variable fragments thereof. More preferably, the antibodies, or fragments thereof, are monoclonal.

According to a second aspect, the present invention provides an immunoassay kit for detecting different FABPs comprising: (i) a multiplexing system according to the first aspect of the invention; and (ii) one or more detection probes capable of binding to said FABPs, said detection probe comprising a detectable label. Preferably, the detection probes are antibodies. Preferably, the detectable label is a radioactive marker, DNA, fluorescent molecule, or an enzyme which converts a substrate such that its absorbance or chemiluminescence signal can be detected.

Preferably, if; i) the detection probe is a B-FABP binding molecule it binds to SEQ ID No 1 or 2; ii) the detection probe is a H-FABP binding molecule it binds to the epitope of SEQ ID No 3 and/ or 4; iii) the detection probe is a E-FABP binding molecule it binds to SEQ ID No 5 or 6: and/ or iv) the detection probe is a L-FABP binding molecule it binds to SEQ ID No 7 or 8. Preferably, the detection probes and the binding molecules according to the first aspect of the invention bind to discrete epitopes.

In a further preferred embodiment, the detection probes are antibodies, or single chain variable fragments thereof

According to a third aspect, the present invention provides an *in vitro* method of detecting or quantifying B-FABP, L-FABP, H-FABP, and E-FABP within a biological sample, comprising bringing the biological sample into contact with the multiplexing system according to the first aspect of the invention, wherein if binding molecules are immobilised to the support substrate of the multiplexing system, the method further comprises the steps of: (i) removing unbound sample; (ii)

contacting the substrate with a detection probe as defined in the second aspect of the invention under conditions that allow the detection probe to bind; and (iii) detecting the binding of the detection probe on the substrate to thereby determine the presence of FABP.

The binding molecule that binds specifically to B-FABP binds to SEQ ID No 1 or 2. The binding molecule that binds specifically the H-FABP binds to SEQ ID No 3 and/ or 4. The binding molecule that binds specifically the E-FABP binds to SEQ ID No 5 or 6. The binding molecule that binds specifically the L-FABP binds to of SEQ ID No 7 or 8. Preferably, the binding molecule is an antibody, or a single chain variable fragment thereof. More preferably, the antibody or fragment thereof is monoclonal.

**Examples**

**Example 1**

**Development of specific FABP assays and cross-reactivity analysis**

The initial stage of assay development is to identify antibodies, antigens and conjugates which can be used for further assay evaluation. Monoclonal antibodies for the specific FABP assays were developed in house. All monoclonal antibodies obtained for a particular FABP assay, for example L-FABP, were immobilised on the biochip surface (capture antibodies) and these same monoclonal antibodies were conjugated to HRP (detection probes).The optimal antibody pair for L-FABP is then selected through rigorous evaluation of performance characteristics.

**Cross-reactivity analysis**

The specificity, expressed as %cross reactivity (%CR) was calculated as outlined below, using L-FABP as an example.

Individual 9.46 ng/ml aliquots of L-FABP were spiked with 4000ng/ml of one of A-FABP, B-FABP, H-FABP, I-FABP, IL-FABP, E-FABP, M-FABP or T-FABP. The concentration of protein captured and detected within each spiked sample was then calculated using a calibration curve. Each sample was performed alongside an unspiked control sample containing the equivalent concentration of L-FABP. The following calculation was then used to calculate %CR:

$$\%CR = \left( \frac{(OC_s - OC_u)}{TC_s} \right) \times 100$$

wherein $OC_s$ is the total protein concentration measured in the spiked sample, $OC_u$ is the total protein concentration measured in the unspiked sample, and $TC_s$ is the total protein concentration of the spiked sample. Cross reactivity results of L-FABP antibody 1 are shown in Table 1.

**Table 1** Cross reactivity results of L-FABP antibody 1, where $TC_s$ is the total assay concentration, $OC_s$ is the protein concentration measured in the spiked sample, $OC_u$ is the measured concentration in the unspiked sample, and %CR is the cross reactivity as defined in the equation above.

| Cross reactant | $TC_s$ (ng.ml$^{-1}$) | $OC_s$ (ng.ml$^{-1}$) | $OC_u$ (ng.ml$^{-1}$) | %CR |
|---|---|---|---|---|
| B-FABP | 4000 | 9.93 | 10.93 | -0.02 |
| H-FABP | 4000 | 9.02 | 10.92 | -0.05 |
| I-FABP | 4000 | 10.65 | 10.59 | 0.00 |
| IL-FABP | 4000 | 10.83 | 9.46 | 0.03 |
| E-FABP | 4000 | 10.57 | 9.46 | 0.03 |
| M-FABP | 4000 | 10.94 | 10.93 | 0.00 |
| T-FABP | 4000 | 10.78 | 11.49 | 0.00 |
| A-FABP | 4000 | 8.88 | 10.92 | -0.05 |

The %CR analysis was performed as outlined above for L-FABP, H-FABP, A-FABP, E-FABP, IL-FABP and B-FABP. The results are shown in Table 2

**Table 2** %CR of capture probes/antibodies raised against L, H, A, E, IL and B FABP antigens. %CR for each was determined against the cross reactants L, I, H, A, E, II, B, M and T-FABP.

| | | Cross reactant | | | | | | | | |
|---|---|---|---|---|---|---|---|---|---|---|
| | | L-FABP | I-FABP | H-FABP | A-FABP | E-FABP | IL-FABP | B-FABP | M-FABP | T-FABP |
| Capture probe | L-FABP | - | <1% | <1% | <1% | <1% | <1% | <1% | <1% | <1% |
| | H-FABP | <1% | <1% | - | <1% | <1% | <1% | <1% | <1% | <1% |
| | A-FABP | <1% | <1% | <1% | - | <1% | <1% | <1% | <1% | <1% |
| | E-FABP | <1% | <1% | <1% | <1% | - | <1% | <1% | <1% | <1% |
| | IL-FABP | <1% | <1% | <1% | <1% | <1% | - | <1% | <1% | <1% |
| | B-FABP | <1% | <1% | <1% | <1% | <1% | <1% | - | <1% | <1% |

## Example 2

### Analysis of antibody binding locations for H, L, B and E-FABP

BSA conjugated peptides (approximately 20 amino acids per peptide) spanning the entire amino acid sequences of Heart, Liver, Brain and Epidermal FABP were generated. Individual BSA conjugated peptides corresponding to a specific FABP family member were immobilised at distinct test regions (DTR) within a Biochip/ discrete test area (DTA). Peptide Biochips specific for Heart, Liver, Brain and Epidermal FABPs were therefore generated. Appropriate controls (negative and positive) were also incorporated into the Biochips. Antibody pairs (herein referred to as antibodies 1 and 2) for each of the FABPs under investigation were conjugated to HRP. Antibodies for each target were incubated with respective Biochips to enable accurate peptide/epitope mapping. Upon imaging, a signal is observed when a specific interaction occurs between an antibody and its target peptide/epitope (Figure 1).

Figure 1 uses as an example an L-FABP Biochip probed with L-FABP antibody 1 and L-FABP antibody 2 to detect the binding epitopes. The identity of the immobilised peptide at each of the DTRs is as follows; 1) Blank, 2) Blank, 3) Blank, 4) Blank, 5) Reference spot, 6) Reference Spot, 7) L-FABP peptide 1-19, 8) L-FABP peptide 17-35, 9) L-FABP peptide 33-51, 10) L-FABP peptide 49-67, 11) L-FABP peptide 65-83, 12) L-FABP peptide 81-98, 13) Blank, 14) L-FABP peptide 96-112, 15) L-FABP peptide 110-127, 16) Blank, 17) BSA, 18) BSA, 19) cTnI peptide-BSA, 20) Full length L-FABP (source 1), 21) Full length L-FABP (source 2), 22) Blank, 23) Reference spot, 24) Blank, 25) Blank.

The antibodies specific for L-FABP react with DTRs 8 and 15, respectively. Therefore, antibody 1 recognises an epitope within amino acids 17-35, whilst antibody 2 recognises an epitope within the L-FABP sequence from amino acids 110-127 (see Figure 2a for graphical representation of the L-FABP antibody 1 and L-FABP antibody 2 binding sites). Note, that two different sources of full length L-FABP are immobilised to DTRs 20 and 21 which are both recognised by the L-FABP antibodies 1 and 2.

The mapped peptides containing the epitopes of L, H, E and B FABP antibodies are shown in Table 3.

**Table 3** Epitopes mapped for Liver, Heart, Epidermal, and Brain FABP antibodies.

| Antibody | Location of epitope sequence | Peptide sequence | SEQ ID NO: |
|---|---|---|---|
| Liver FABP 1 | 17-35 | AFMKAIGLPEELIQKGK DI | 7 |
| Liver FABP 2 | 110-127 | TNTMTLGDIVFKRISKRI | 8 |
| Heart FABP 1 | 1-19 | MVDAFLGTWKLVDSKN FDD | 3 |
| Heart FABP 2 | 1-19 & 81-99 | MVDAFLGTWKLVDSKN FDD & VKSIVTLDGGKLVHLQK WD * | 3 4 |
| Epidermal FABP 1 | 17-35 | GFDEYMKELGVGIALR KM | 5 |
| Epidermal FABP 2 | 1-19 | MATVQQLEGRWRLVD SKGF | 6 |
| Brain FABP 1 | 65-83 | LGEEFDETTADDRNCK SV | 1 |
| Brain FABP 2 | 113-132 | DGKMVMTLTFGDVVAV RHYEKA | 2 |

* Heart FABP antibody 2 is binding two peptides. The conformation of the protein was investigated and it appears that the two peptides are in close proximity to each other in the context of the predicted 3D structure. It is therefore feasible that both peptides cumulatively contain the binding epitope for this antibody.

Also described is the following:

1. A multiplexing system comprising a support substrate having immobilised thereon one of the following proteins: E-FABP, B-FABP, IL-FABP, I-FABP, M-FABP, A-FABP, H-FABP, L-FABP or T-FABP, or a peptide fragment thereof, or a binding molecule, said binding molecule binding specifically to one of said proteins.

2. The multiplexing system of embodiment 1, comprising a plurality of the proteins, peptide fragments, or binding molecules defined in embodiment 1.

3. The multiplexing system of embodiment 1 or embodiment 2, comprising two or more of the binding molecules or proteins, or peptide fragments thereof, preferably 3 or more of the binding molecules or proteins, or peptide fragments thereof, preferably 4 or more of the binding molecules or proteins, or peptide fragments thereof, defined in embodiment 1, wherein each binding molecule, protein, or peptide fragment thereof is immobilised to a separate support substrate or on discrete areas of the same support substrate.

4. The multiplexing system of any preceding embodiment, wherein the support substrate is a bead or microparticle or is a planar surface comprising a plurality of discrete reaction zones,
preferably wherein each binding molecule or protein, or peptide fragment thereof, is immobilised upon a separate bead or microparticle or is immobilised upon a discrete reaction zone upon a planar substrate.

5. The multiplexing system of any preceding embodiment, comprising;

   i. the binding molecule that binds specifically to B-FABP and additionally one or more of the binding molecules each of which bind specifically to a single FABP selected from A-FABP, E-FABP, or H-FABP; or
   ii. the binding molecule that binds specifically to E-FABP and additionally one or more of the binding molecules each of which bind specifically to a single FABP selected from A-FABP or H-FABP; or
   iii. B-FABP, or a peptide fragment thereof, and additionally one or more of A-FABP, E-FABP, or H-FABP, or peptide fragments thereof; or
   iv. E-FABP, or a peptide fragment thereof, and additionally one or more of A-FABP, or HFABP, or peptide fragments thereof.

6. The multiplexing system of any preceding embodiment, wherein the multiplexing system comprises;

   four binding molecules each of which is specific for a single different FABP selected from E-FABP, H-FABP, A-FABP, and B-FABP;
   preferably wherein one or more further binding molecules are present, each of which bind specifically to a single different FABP selected from IL-FABP, I-FABP, L-FABP, M-FABP, and T-FABP,
   preferably wherein binding molecules, each of which bind specifically to a single FABP selected from IL-FABP, I-FABP, L-FABP, M-FABP, and T-FABP, are present.

7. The multiplexing system of any of embodiments 1 to 5, wherein the multiplexing system comprises:

   E-FABP, H-FABP, B-FABP, and A-FABP, or peptide fragments thereof,
   preferably wherein one or more of IL-FABP, I-FABP, L-FABP, M-FABP, and T-FABP, or peptide fragments thereof, are present,
   preferably wherein IL-FABP, I-FABP, L-FABP, M-FABP, and T-FABP, or peptide fragments thereof, are present.

8. The multiplexing system of any of embodiments 1 to 6, wherein the binding molecules specific to each FABP display a cross-reactivity of less than 5%, preferably less than 1%, to the other FABP isoforms.

9. The multiplexing system of embodiment 8, wherein;

   i. the B-FABP binding molecule binds to SEQ ID NO 1 or 2;
   ii. the H-FABP binding molecule binds to SEQ ID NO 3 and/ or 4;
   iii. the E-FABP binding molecule binds to SEQ ID NO 5 or 6;
   iv. the L-FABP binding molecule binds to SEQ ID NO 7 or 8,

      preferably wherein each binding molecule binds to at least 3 amino acids, preferably 3 to 7 amino acids, most preferably 3 to 5 amino acids from the corresponding SEQ-IDs,
      preferably wherein the binding molecules are antibodies, or single chain variable fragments thereof,
      preferably wherein the antibodies are monoclonal.

10. An immunoassay kit for detecting two or more different FABPs comprising;

   i. a multiplexing system according to any preceding claim; and
   ii. one or more detection probes capable of binding to said FABPs, said detection probe comprising a detectable label preferably wherein the detectable label is a radioactive marker, DNA, fluorescent molecule, or an enzyme which converts a substrate such that its absorbance or chemilumescence signal can be detected.

11. A kit according to embodiment 10, wherein if;

   i. detection probe is a B-FABP binding molecule, or fragment thereof, it binds to SEQ ID NO 1 or 2;
   ii. detection probe is a H-FABP binding molecule, or fragment thereof, it binds to SEQ ID NO 3 and/ or 4;
   iii. detection probe is an E-FABP binding molecule, or fragment thereof, it binds to SEQ ID NO 5 or 6; and
   iv. detection probe is a L-FABP binding molecule, or fragment thereof, it binds to SEQ ID NO 7 or 8,

      preferably wherein said detection probe and the binding molecules defined in any of embodiments 1-9 bind to different epitopes

preferably wherein said detection probes are antibodies, or single chain variable fragments thereof.

12. An *in vitro* method of detecting A-FABP, B-FABP, L-FABP, I-FABP, IL-FABP, H-FABP, T-FABP, M-FABP and E-FABP, or any combination thereof, within a biological sample, comprising bringing the biological sample into contact with the multiplexing system defined in any of embodiments 1 to 9,

wherein if binding molecules are immobilised to the support substrate of the multiplexing system, the method further comprises the steps of;

i. washing the substrate to remove unbound sample;
ii. contacting the substrate with a detection probe as defined in embodiment 10 or 11, under conditions to allow the detection probe to bind to any bound FABP; and
iii. detecting the binding of the detection probe on the substrate to thereby determine the presence of FABP,

or wherein proteins, or peptide fragments thereof, are immobilised to the support substrate of the multiplexing system, the method further comprises the steps of;

i. contacting the biological sample with a detection probe as defined in embodiment 10 or 11, before, at the same time or after the biological sample is contacted to the multiplexing system;
ii. incubating the biological sample and the detection probe under conditions that allow the detection probe to bind to FABP present in the biological sample; and
iii. detecting the binding of the detection probe on the multiplexing system to thereby determine the absence of FABP.

13. A method according to embodiment 12, wherein the presence of two or more FABPs are assayed for in a biological sample and at least one of the FABPs assayed for is E-FABP and/or B-FABP, and at least one additional FABP assayed for is selected from H-FABP, A-FABP, L-FABP, IL-FABP, M-FABP, I-FABP and/or T-FABP,

preferably wherein the additional FABP is H-FABP and/or A-FABP,
preferably wherein the biological sample is blood.

14. A binding molecule that binds one of H-FABP, E-FABP, B-FABP, A-FABP, II-FABP, T-FABP, L-FABP, M-FABP or I-FABP and with less than 5% cross-reactivity, preferably less than 1% cross-reactivity, to the other FABP isoforms.

15. A binding molecule according to embodiment 14, wherein;

i. the binding molecule that binds B-FABP binds to SEQ ID No 1 or No 2;
ii. the binding molecule that binds H-FABP binds to SEQ ID No 3 and/ or No 4;
iii. the binding molecule that binds E-FABP binds to SEQ ID No 5 or No 6; or
iv. the binding molecule that binds L-FABP binds to SEQ ID No 7 or No 8.

preferably wherein the binding molecule is an antibody, or single chain variant thereof,
preferably wherein the antibody, or fragment thereof is monoclonal

16. The use of a binding molecule defined in either of embodiments 14 or 15 for determining the presence of B-FABP, H-FABP, E-FABP or L-FABP within a sample.

17. A method to aid detection of tissue or cellular damage in a subject, comprising performing the method as defined in embodiment 12, wherein the combination of FABPs detected within the biological sample is indicative of the site or sites of tissue or cellular damage within the subject.

**Claims**

1. A multiplexing system comprising a support substrate having immobilised thereon a binding molecule for each of the following, Brain-Fatty Acid Binding Protein (B-FABP), Liver-Fatty Acid Binding Protein (L-FABP), Heart-Fatty Acid Binding Protein (H-FABP) and Epidermal-Fatty Acid Binding Protein (E-FABP), wherein the binding molecule for each binds specifically to said Fatty Acid Binding Protein (FABP) at the following regions; B-FABP at the region defined by any of SEQ ID Nos. 1 (LGEEFDETTADDRNCKSV) and 2 (DGKMVMTLTFGDVVAVRHYEKA), L-FABP at the region defined by any of SEQ ID Nos. 7 (AFMKAIGLPEELIQKGKDI) and 8 (TNTMTLGDIVFKRISKRI), H-FABP at any of the region defined by any of SEQ ID Nos. 3 (MVDAFLGTWKLVDSKNFDD) and 4 (VKSIVTLDGGKLVHLQKWD), E-FABP at the region defined by any of SEQ ID Nos. 5 (GFDEYMKELGVGIALRKM) and 6 (MATVQQLEGRWRLVDSKGF), wherein the binding molecules specific to each FABP display a cross-reactivity of less than 5% to the other isoforms.

2. The multiplexing system according to claim 1 further comprising a binding molecule for one or more of IL-FABP, I-FABP, M-FABP, A-FABP, or T-FABP.

3. The multiplexing system of claim 1 or claim 2, wherein the support substrate is a bead or microparticle or is a planar surface comprising a plurality of discrete reaction zones,
preferably wherein each different binding molecule or protein, is immobilised upon a separate bead or microparticle or is immobilised upon a discrete reaction zone upon a planar substrate.

4. The multiplexing system of claim 1, wherein the cross-reactivity is less than 1% to the other FABP isoforms.

5. The multiplexing system of any preceding claim, wherein;
the binding molecules are antibodies, or single chain variable fragments thereof.

6. An immunoassay kit for detecting different FABPs comprising;

   i. a multiplexing system according to any preceding claim; and
   ii. one or more detection probes capable of binding to said FABPs, said detection probe comprising a detectable label preferably wherein the detectable label is a radioactive marker, DNA, fluorescent molecule, or an enzyme which converts a substrate such that its absorbance or chemilumescence signal can be detected.

7. The kit according to claim 6, wherein;
said detection probes are antibodies, or single chain variable fragments thereof.

8. An *in vitro* method of detecting or quantifying B-FABP, L-FABP, H-FABP, and E-FABP, within a biological sample, comprising bringing the biological sample into contact with the multiplexing system defined in any of claims 1 to 5, wherein if binding molecules are immobilised to the support substrate of the multiplexing system, the method further comprises the steps of;

   i. removing unbound sample;
   ii. contacting the substrate with a detection probe as defined in claim 6 or 7, under conditions to allow the detection probe to bind to any bound FABP; and
   iii. detecting the binding of the detection probe on the substrate to thereby determine the presence of FABP.

9. A method according to claim 8, wherein the presence of FABPs are assayed for in a biological sample wherein the biological sample is blood.

**Patentansprüche**

1. Multiplexsystem, umfassend ein Trägersubstrat, auf dem ein Bindemolekül für jedes der Folgenden immobilisiert ist, Fettsäurebindeprotein des Gehirns (B-FABP), Fettsäurebindeprotein der Leber (L-FABP), Fettsäurebindeprotein des Herzens (H-FABP) und epidermales Fettsäurebindeprotein (E-FABP), wobei sich das Bindemolekül für jedes spezifisch an das Fettsäurebindeprotein (FABP) an den folgenden Regionen bindet; B-FABP an der Region, die durch eine beliebige von SEQ ID Nr. 1 (LGEEFDETTADDRNCKSV) und 2 (DGKMVMTLTFGDVVAVRHYEKA) definiert ist, L-FABP an der Region, die durch eine beliebige von SEQ ID Nr. 7 (AFMKAIGLPEELIQKGKDI) und 8 (TNTMTLGDIVFKRISKRI) definiert ist, H-FABP an der Region, die durch eine beliebige von SEQ ID Nr. 3 (MVDAFLGTWKLVDSKNFDD) und 4 (VKSIVTLDGGKLVHLQKWD) definiert ist, E- FABP an der Region, die durch eine beliebige von SEQ ID Nr. 5 (GFDEYMKELGVGIALRKM) und 6 (MATVQQLEGRWRLVDSKGF) definiert ist, wobei die Bindemoleküle, die für jedes FABP spezifisch sind, eine Kreuzreaktivität von weniger als 5% anderen Isoformen gegenüber aufzeigen.

**EP 3 479 124 B1**

2. Multiplexsystem nach Anspruch 1, weiter umfassend ein Bindemolekül für eines oder mehrere von IL-FABP, I-FABP, M-FABP, A-FABP oder T-FABP.

3. Multiplexsystem nach Anspruch 1 oder Anspruch 2, wobei das Trägersubstrat ein Kügelchen oder Kleinstteilchen ist oder eine ebene Oberfläche ist, die eine Vielzahl von diskreten Reaktionsbereichen umfasst, wobei vorzugsweise jedes unterschiedliche Bindemolekül oder -protein auf einem separaten Kügelchen oder Kleinstteilchen immobilisiert ist oder auf einem diskreten Reaktionsbereich auf einem ebenen Substrat immobilisiert ist.

4. Multiplexsystem nach Anspruch 1, wobei die Kreuzreaktivität anderen FABP-Isoformen gegenüber weniger als 1% beträgt.

5. Multiplexsystem nach einem vorstehenden Anspruch, wobei; die Bindemoleküle Antikörper oder einkettige variable Fragmente davon sind.

6. Immunoassay-Kit zum Erfassen unterschiedlicher FABPs, umfassend;

   i. ein Multiplexsystem nach einem vorstehenden Anspruch; und
   ii. eine oder mehrere Erfassungssonden, die imstande sind, sich an die FABPs zu binden, wobei die Erfassungssonde eine erfassbare Kennzeichnung umfasst,

   wobei die erfassbare Kennzeichnung vorzugsweise eine radioaktive Markierung, DNA, ein fluoreszierendes Molekül oder ein Enzym ist, das ein Substrat so umwandelt, dass dessen Absorptionsfähigkeit oder Chemilumineszenzsignal erfasst werden kann.

7. Kit nach Anspruch 6, wobei; die Erfassungssonden Antikörper oder einkettige variable Fragmente davon sind.

8. In-vitro-Verfahren zum Erfassen oder Quantifizieren von B-FABP, L-FABP, H-FABP und E-FABP in einer biologischen Probe, umfassend Setzen der biologischen Probe in Kontakt mit dem in einem der Ansprüche 1 bis 5 definierten Multiplexsystem, wobei, wenn die Bindemoleküle am Trägersubstrat des Multiplexsystems immobilisiert sind, das Verfahren weiter die folgenden Schritte umfasst;

   i. Entfernen der ungebundenen Probe;
   ii. Kontaktieren des Substrats mit einer Erfassungssonde wie in Anspruch 6 oder 7 definiert unter Bedingungen, die ermöglichen, dass die Erfassungssonde sich an ein beliebiges FABP bindet; und
   iii. Erfassen der Bindung der Erfassungsprobe an das Substrat, um dadurch das Vorhandensein von FABP zu bestimmen.

9. Verfahren nach Anspruch 8, wobei das Vorhandensein von FABPs in einer biologischen Probe geprüft wird, wobei die biologische Probe Blut ist.

**Revendications**

1. Système de multiplexage comprenant un substrat support sur lequel est immobilisée une molécule de liaison pour chacune des protéines suivantes, une protéine de liaison aux acides gras du cerveau (B-FABP), une protéine de liaison aux acides gras du foie (L-FABP), une protéine de liaison aux acides gras du cœur (H-FABP) et une protéine de liaison aux acides gras épidermiques (E-FABP), dans lequel la molécule de liaison pour chacun se lie spécifiquement à ladite protéine de liaison aux acides gras (FABP) au niveau des régions suivantes ; la B-FABP au niveau de la région définie par l'un quelconque de SEQ ID Nos. 1 (LGEEFDETTADDRNCKSV) et 2 (DGKMVMTLTFGDVVAVRHYEKA), la L-FABP au niveau de la région définie par l'un quelconque de SEQ ID Nos. 7 (AFMKAIGLPEELIQKGKDI) et 8 (TNTMTLGDIVFKRISKRI), la H-FABP au niveau de l'une quelconque de la région définie par l'un quelconque de SEQ ID Nos. 3 (MVDAFLGTWKLVDSKNFDD) et 4 (VKSIVTLDGGKLVHLQKWD), la E- FABP au niveau de la région définie par l'un quelconque de SEQ ID Nos. 5 (GFDEYMKELGVGIALRKM) et 6 (MATVQQLEGRWRLVDSKGF), dans lequel les molécules de liaison spécifiques à chaque FABP affichent une réactivité croisée inférieure à 5 % par rapport aux autres isoformes.

18

**2.** Système de multiplexage selon la revendication 1, comprenant en outre une molécule de liaison pour une ou plusieurs de IL-FABP, I-FABP, M-FABP, A-FABP ou T-FABP.

**3.** Système de multiplexage selon la revendication 1 ou la revendication 2, dans lequel le substrat support est une bille ou une microparticule ou est une surface plane comprenant une pluralité de zones de réaction discrètes,
de préférence dans lequel chaque molécule, ou protéine, de liaison différente est immobilisée sur une perle, ou microparticule, distincte ou est immobilisée sur une zone de réaction discrète sur un substrat plan.

**4.** Système de multiplexage selon la revendication 1, dans lequel la réactivité croisée est inférieure à 1 % par rapport aux autres isoformes de FABP.

**5.** Système de multiplexage selon une quelconque revendication précédente, dans lequel :
les molécules de liaison sont des anticorps ou des fragments variables à chaîne unique de ceux-ci.

**6.** Kit d'immuno-essai pour détecter différentes FABP comprenant :

    i. un système de multiplexage selon une quelconque revendication précédente ; et
    ii. une ou plusieurs sondes de détection qui permettent une liaison auxdites FABP, ladite sonde de détection comprenant une étiquette détectable

de préférence dans lequel l'étiquette détectable est un marqueur radioactif, un ADN, une molécule fluorescente ou une enzyme qui convertit un substrat de telle sorte que son signal d'absorbance ou de chimilumescence puisse être détecté.

**7.** Kit selon la revendication 6, dans lequel :
lesdites sondes de détection sont des anticorps ou des fragments variables à chaîne unique de ceux-ci.

**8.** Procédé *in vitro* de détection ou de quantification des B-FABP, L-FABP, H-FABP et E-FABP, dans un échantillon biologique, comprenant la mise en contact de l'échantillon biologique avec le système de multiplexage défini dans l'une quelconque des revendications 1 à 5,
dans lequel, si des molécules de liaison sont immobilisées sur le substrat support du système de multiplexage, le procédé comprend en outre les étapes consistant à :

    i. éliminer un échantillon non lié ;
    ii. mettre en contact le substrat avec une sonde de détection telle que définie dans la revendication 6 ou 7, dans des conditions pour permettre à la sonde de détection d'être liée à n'importe quelle FABP liée< ; et
    iii. détecter la liaison de la sonde de détection sur le substrat pour déterminer, de ce fait, la présence d'une FABP.

**9.** Procédé selon la revendication 8, dans lequel la présence de FABP est analysée dans un échantillon biologique, dans lequel l'échantillon biologique est du sang.

Numbering Template         L-FABP antibody 1          L-FABP antibody 2

| 5 | 4 | 3 | 2 | 1 |
|---|---|---|---|---|
| 10 | 9 | 8 | 7 | 6 |
| 15 | 14 | 13 | 12 | 11 |
| 20 | 19 | 18 | 17 | 16 |
| 25 | 24 | 23 | 22 | 21 |

**Figure 1**

**Figure 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2283360 A
- WO 2009139632 A
- GB 2324866 A